# EUROPEAN PATENT SPECIFICATION

(11) **EP 1 653 912 B1**
(45) Date of publication and mention of the grant of the patent: **05.10.2011**
(21) Application number: 04780361.4
(22) Date of filing: 05.08.2004
(51) Int. Cl.: A61K 39/39, A61K 38/19

(54) **VACCINE IMMUNOTHERAPY FOR IMMUNE SUPPRESSED PATIENTS**
VAKZINE-IMMUNTHERAPIE FÜR IMMUNGESCHWÄCHTE PATIENTEN
IMMUNOTHERAPIE VACCINALE POUR PATIENTS IMMUNODEPRIMES

(30) Priority: 08.08.2003 US 637869
(43) Date of publication of application: 10.05.2006
(73) Proprietor: IRX Therapeutics, Inc., New York, NY 10019 (US)
(72) Inventor: HADDEN, John, W., Cold Spring Harbor, NY 11724 (US)
(74) Representative: Austin, Hedley William
(86) International application number: PCT/US2004/025518
(87) International publication number: WO 2005/025494

(56) References cited:
- WO-A-02/34119
- WO-A-03/035004
- US-A- 5 632 983
- DUEÑAS-GONZALEZ A ET AL: "A pilot study of perilymphatic leukocyte cytokine mixture (IRX-2) as neoadjuvant treatment for early stage cervical carcinoma." INTERNATIONAL IMMUNOPHARMACOLOGY JUN 2002, vol. 2, no. 7, June 2002 (2002-06), pages 1007-1016, XP002442178 ISSN: 1567-5769
- BRANDWEIN H: "Session V: Strategies for immunization - IRX-2: A natural cytokine stimulant for cancer vaccines" CANCER IMMUNOLOGY, IMMUNOTHERAPY 2003 GERMANY, vol. 52, no. SUPPL. 1, March 2003 (2003-03), pages S17-S18+S30, XP002442179 ISSN: 0340-7004
- NAYLOR PAUL H ET AL: "T cell targeted immune enhancement yields effective T cell adjuvants." INTERNATIONAL IMMUNOPHARMACOLOGY AUG 2003, vol. 3, no. 8, August 2003 (2003-08), pages 1205-1215, XP002442180 ISSN: 1567-5769
- MENESES ABELARDO ET AL: "Lymph node histology in head and neck cancer: impact of immunotherapy with IRX-2." INTERNATIONAL IMMUNOPHARMACOLOGY AUG 2003, vol. 3, no. 8, August 2003 (2003-08), pages 1083-1091, XP002442181 ISSN: 1567-5769
- HADDEN J ET AL: "A trial of IRX-2 in patients with squamous cell carcinomas of the head and neck." INTERNATIONAL IMMUNOPHARMACOLOGY AUG 2003, vol. 3, no. 8, August 2003 (2003-08), pages 1073-1081, XP002341580 ISSN: 1567-5769

## Description

The present invention relates to vaccine therapy for cancer patients

It has become increasingly apparent that human cancers have antigens, which if reacted upon by the host's immune systems, lead to tumour regression. These antigens have been defined by both serological and cellular immune approaches. This has lead to the definition of both B and T cell epitopes (Sahin U, et al, Curr Opin Immunol 9:709-715, 1997; Van der Eynde, B, et al. Curr Opin Immunol 9:684-693, 1997; Wang RF, et al., Immunologic Reviews 170:85-100, 1999). Based upon these results, it has become a goal of cancer immunotherapists to induce regressions of tumours. However, historically, successful efforts have been sporadic and generally minor in frequency and magnitude.

A fundamental problem in the effort to immunise cancer patients is that the tumour-bearing state is associated with immunosuppressive mechanisms derived from both the tumour and from the host's disturbed immune system (Kavanaugh D Y, et al, Hematol-Oncol Clinics of North Amer 10(4):927-951, 1996), thereby making immunisation difficult and until now impossible on a consistent basis. Immune suppression or depletion involves a reduced capacity of the immune system to respond. Such suppression can be drug or disease induced. The condition can be drug induced by treatment, virus induced as in AIDS, or induced by a disease state such as cancer. The immune system in this condition is effectively turned off.

A variety of tumour immunisation strategies have been developed. However, all such strategies are complex and deviate significantly from the conventional immunisation strategies used for infectious diseases (Weber J. Tumor, Medscape Anthology 3:2, 2000). One such tumour immunization strategy involves a material sold under the trade mark Theratope, a Sialyl TN polysaccharide mucin antigen conjugated with keyhole limpet hemocyanine and administered with Detox mycobacterium adjuvant (Detox is a trade mark) and low dose cyclophosphamide (Maclean G D, et al, J Immunother Emphasis Tumor Immunol 19(4):309-316, 1996). However, use of this vaccine in patients with metastatic breast and ovarian cancer has yielded major clinical responses in a low percentage of patients. A major response means greater than 50% tumour reduction.

Gene therapy has also been attempted using an adenovirus construct as an expression vector for genes expressing Papilloma virus peptide 16 has been used for immunization or patients with cervical cancer and has yielded major clinical responses in a low percentage of patients (Borysiewickz L K, et al, Lancet 347:1524-1527, 1996).

Dendritic cell mediated therapy has also been attempted, in which dendritic cells were pulsed with oligopeptide fragments of prostate specific antigens (PSA). Prostate specific membrane antigen (PSMA) has been used in patients with metastatic prostate cancer with major clinical responses in a low percentage of patients (Sanda M G, et al, Urology 52:2, 1999; Murphy GP, et al, The Prostate. 38:43-78, 1999).

Additionally, autologous tumours have been used with low dose cyclophosphamide and BCG to immunize cancer patients with malignant melanoma. However, few clinical responses were reported (Mastrangelo M J, et al, Seminars in Oncology 23(6):773-781, 1996). Another strategy attempted included using MAGE antigens with a variety of vaccine adjuvants. Again, this has yielded few, if any, responses in patients with malignant melanoma.

US Patents 5503841; 5800810; 6060068; 5643565 and 5100664 (all in the name of Doyle) disclose methods of enhancing the immune response in patients using Interleukin 2-(IL-2). This method is disclosed for use in response to infectious diseases and primarily functions using antigens known to be immunogenic

As disclosed above, the treatment of cancer is known to require different approaches. To date, treatment with IL-2 has shown minor effects in two cancers, renal cell and malignant melanoma (response rates less than 20%). It is generally considered ineffective in head and neck squamous cell cancer (H&NSCC); in cervical cancer and in prostate cancer. Hence, it is not approved for these uses. It would therefore not be obvious based on the method of the above patents to use of small peptides in the treatment of cancer.

It is important to contrast prevention with known "classic" antigens of complex structure and high molecular weights in healthy patients vs. treatment (generally unsuccessful) with tumour antigens or peptides (general unsuccessful) in immunosupressed patients (generally unsuccessful). The first is easy and our current viral vaccines attest to their efficacy. The latter is nearly impossible on a routine basis despite 30 years of intense effort.

Immunisation has now been proposed with endogenous peptide processed and presented by dendritic cells or endogenously administered to an environment (lymph node) where dendritic cells have been prepared and can present them to T-cells effectively. This was once considered to be an insurmountable challenge. Peptides are much too small to be effective immunogens; their half life is short they are often non-mutated self antigens to which the patient is immunologically tolerant and gaining a response is tantamount to inducing auto immunity.

In several of the above strategies, cellular and/or tumoral immunity to tumour-associated antigens has been induced (Weber J. Tumor, Medscape Anthology 3:2, 2000; Maclean G D, et al, J Immunother Emphasis Tumor Immunol 19(4):309-316,1996; Borysiewickz L K, et al, Lancet 347:1524-1527, 1996; Sanda M G, et al, Urology 52:2,1999). This is especially so in association with tumour regression. Nevertheless, the success rate of such treatments is negligible and inconsistent (<30%).

NCM, a non-recombinant cytokine mixture, is now known to be active in unblocking immunisation at a regional lymph node. NCM, as defined in US Patents 5632983 and 5698194, is (in brief) prepared in the continuous presence of a 4-aminoquinolone antibiotic and with the continuous or pulsed presence of a mitogen, which in the preferred embodiment is PHA.

WO02/34119 and WO03/035004 both disclose the use of such a natural cytokine mixture (NCM), which includes the cytokines IL-1, IL-2, IL-6, IL-8, IL-12, IFN-gamma, TNF alpha, G-CSF and GM-CSF, for the treatment of cancer patients, generally by injection, resulting in maturation of immature dendritic cells in regional lymph nodes. These documents give a great deal of information concerning how the NCM may be administered (typically by injection around lymphatics that drain into lymph nodes), in some embodiments together with thymosin α1. The NCM may further be administered together with cyclophosphamide (CY) and a non-steroidal antiinflammatory drug (NSAID) such as indomethacin (INDO).

Detailed instructions and examples are given in both documents of modes of administration and modes of action to immunosuppressed patients, for example, together with an "adjuvant" which can enhance the immune response to a particular antigen, and together with a n exogenous or endogenous tumour associated antigen..

Example 1 of WO03/035004 discloses the pre-testing of patients with a skin test using NCM, but therapy with NCM is only indicated after a positive result showing responsiveness to NCM. In other words, the use of NCM is contraindicated after such a negative result is obtained. Further analysis of the clinical, pathological and survival data of the study of that Example offers more insights as it relates to immumisation of cancer patients to their own autologous tumour antigens and the resulting immune regression of their tumours.

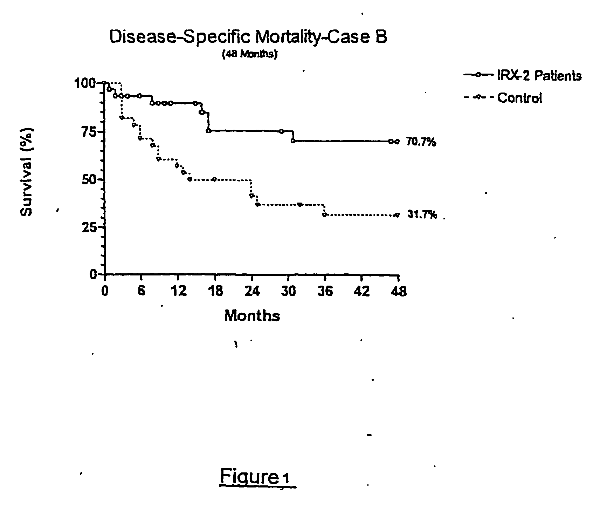

In the accompanying drawings, Figure 1, which is a graph illustrating the survival percentage of treated patients at 48 months, shows that treatment of patients with NCM (referred to as IRX-2) is associated with increased survival at 48 months (p < 0.01).

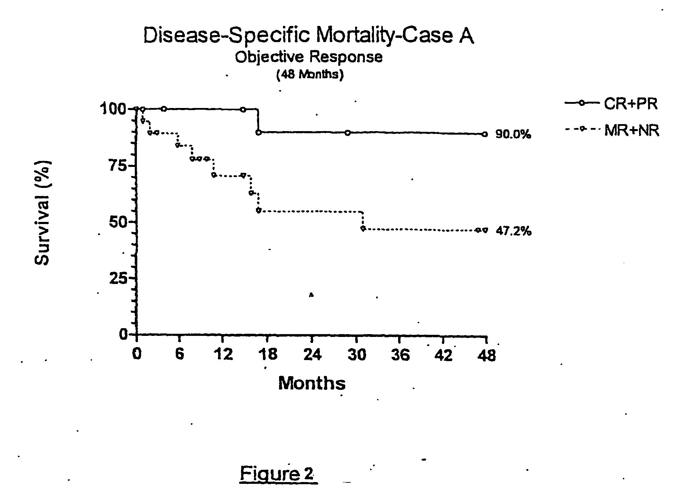

Figure 2 is a graph illustrating the survival of complete and partial responders compared to minor and non-responders, as will be explained herein. The figure shows that clinical responses determine survival, in that patients with complete response (CR) and partial response (PR) (>50% tumour reduction) have better survival rate than those with minor responses (MR) (<50%, but >24% tumour reduction) or no response (NR) (<25%)(p<0.01).

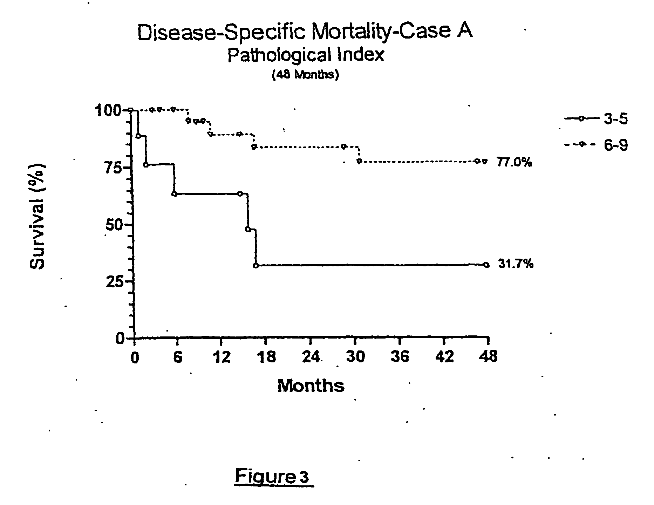

Figure 3, which is a graph illustrating the relationship of pathology index to survival, shows that patients with stronger pathological responses (index of 6 to 9) have better survival rate than those with weaker pathological responses (<6) (p<0.02).

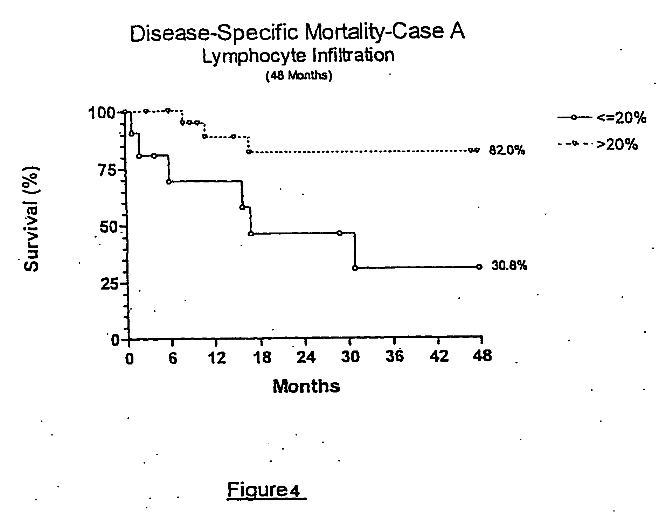

Figure 4, which is a graph showing the relationship of lymphocyte infiltration to survival shows that lymphoid infiltration into the tumour as a single variable predicts survival.

Figure 5 is a graph illustrating the survival percentage (dose response) of treated patients at 24 months, wherein "x" is equal to about 100 IU/mL of IL-2.

Chi Square analysis of the relationship of clinical response to the pathological response in the above figures shows a highly significant relationship (p<0.01) indicating that the two are coordinately related to each other as well as to survival and thus provide a statistical triangulation of the data interrelating clinical responses, immune regression parameters, and survival. Such relationships have never been shown for immunotherapy of a human cancer.

This has led to the surprising finding that it can be advantageous to administer the NCM to a cancer patient who shows a negative skin test result to an NCM skin test.

Accordingly, the present invention comprises Use of a natural cytokine mixture (NCM) including the cytokines L-1, IL-2, IL-6, IL-8, IL-12, IFN-gamma, TNF-alpha, GM-CSF and G-CSF (including recombinants thereof), for immunotherapy of a cancer patient in a dosage regime comprising
(i) application of said natural cytokine mixture in a skin test amount to a cancer patient such that said application shows either a positive or negative clinical response to said skin test within 24 hours, and
(ii) subsequent intracutaneous administration of said natural cytokine mixture to a cancer patient who shows said negative clinical response to said application in step (i).

Preferred features of the present invention are set out in the subsidiary claims.

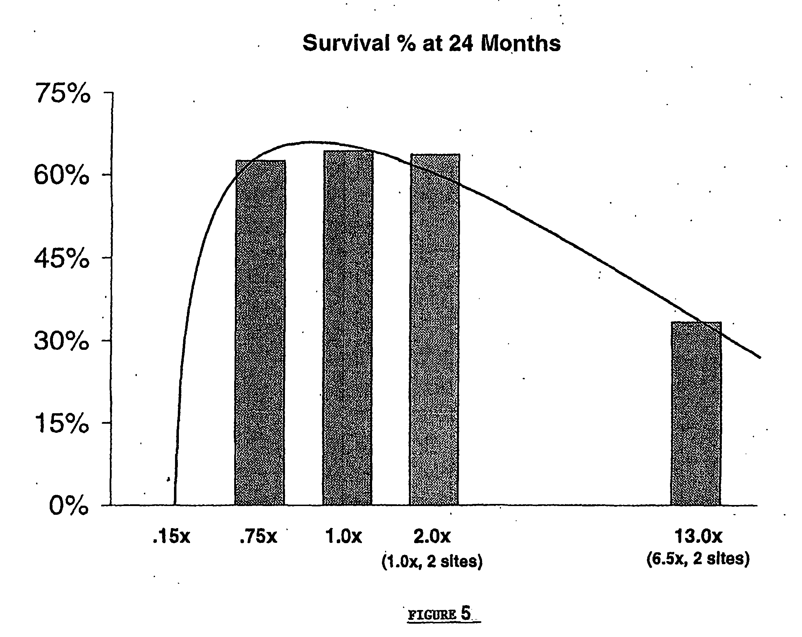

The abovementioned Figure 5 illustrates a series of doses of NCM on overall survival for 24 months. An optimal impact on survival is over the range 100 to 233 equivalents as this amount has optimal impact on survival. No effect was observed at about 16 units and less effect at about 1320 units.

As previously described in the abovementioned WO02/34119 and WO03/035004, the NCM for use according to the invention is preferably for injection around lymphatics that drain into lymph nodes regional to a tumour.

The NCM is preferably co-delivered with CY and an NSAID. The NSAID of choice is indomethacin (INDO). However, ibuprofen, celecoxib, rofecoxib and other CoxII inhibitors can also be used as the NSAID. Side effects of NSAIDs should be aggressively treated with proton inhibitors and a prostaglandin E analog.

Zinc and multi-vitamins are useful agents to help restore T cell immunity, possibly including the addition of selenium. Preferable dosing of zinc is 50 to 75 mg. A standard multivitamin can be administered. The zinc can be an available gluconate.

The present invention will now be further illustrated with reference to the following worked example.

### EXAMPLE

As mentioned above, WO 03/035004 suggests that patients with a negative intradermal skin test to the NCM might show poor clinical responses.

However, we have now accumulated a series of skin test negative patients and find that they show changes similar to the CY and INDO combination without significant NCM. Ten patients had negative skin tests (including four from Juarez Hospital) with NCM (i.e. unresponsive to NCM) and were treated with the NCM plus CY and INDO. These patients had poor clinical responses, smaller tumour reduction and fragmentation, and poor survival (20%) (See Table 1). These observations corroborate the conclusion that INDO and CY have marked activity without NCM.

Importantly they confirm that the skin test is critical for predicting the emphatic clinical and pathological responses that relate to improved survival. In addition, a negative skin test predicts the failure of patients to respond to surgery with or without radiotherapy. The NCM skin test can be usefully employed to predict therapeutic outcome in H & N SCC. Previously, skin testing with dinitrochlorobenzene (DNCB) showed prognostic significance in H & N SCC, but due to the cumbersome procedure requiring sensitization, it is not used clinically anymore. The NCM skin test offers a convenient twenty-four hour test. Interestingly, these patients divided into two groups. In one group, Table I(b), the responses were especially poor with no survivors. In the other group, Table I(a), these patients converted the negative NCM test into a positive following treatment and showed clinical and pathological responses and survival similar to on-protocol patients.

One of these patients had a tumor considered inoperable and was shown to convert the negative test to positive and allowed a second treatment to clinically reduce the tumour and by pathological criteria and to allow prolonged survival following surgery (> 7 years). This pretreatment of skin test negative patients with NCM can increase response rates. NCM plus thymosin α₁ can also be predicted to work (See, United States Published Application No. 20030124136). The negative NCM skin test reflects a monocyte defect and treatment with monocyte-active cytokines in natural or recombinant form would be predicted to be useful singly or in combination thereof. These include, but are not limited to, GM-CSF, M-CSF, IFN-δ, IL-1, IL-6, II-8, IL-12 and others.

**Table I: Negative NCM Skin Test Patients**

| Table I(a) | | | | | | | | | |
|---|---|---|---|---|---|---|---|---|---|
| Patient No. | Patient Initials | Tumor % | Solid % | Frag. % | Stroma % | Lymph. % | Absolute Tumor Reduction | Subj. Resp. | Status |
| Negative NCM Skin Test Changed to Positive | | | | | | | | | |
| 13 | ANA | 48 | 15 | 33 | 16 | 36 | 42 | PR | Alive > 24 Mos. |
| 15 | ICV | 70 | 63 | 7 | 6 | 24 | 5 | MR | Alive > 24 Mos. |
| 22 | JMM | 50 | 10 | 40 | 10 | 40 | 30 | PR | Died without Disease 9 Mos. |
| 27 | MVR | 70 | 28 | 42 | 12 | 18 | 10 | PR | Lost to Follow-up |
| | Mean | 60 | 29 | 31 | 11 | 30 | 22 | | |
| | SD | 12 | 24 | 16 | 4 | 10 | 17 | | |
| | | | | | | | | | |

| Table I(b) | | | | | | | | | |
|---|---|---|---|---|---|---|---|---|---|
| Negative NCM Skin Test | | | | | | | | | |
| 29 | JISM | 80 | 80 | 0 | 10 | 10 | 0 | NR | Died of Disease < 1 Year |
| 30 | AGM | 80 | 48 | 32 | 10 | 10 | 0 | NR | Died of Disease < 1 Year |
| 35 | NGS* | 70 | 70 | 0 | 0 | 30 | 0 | NR | Died of Disease < 1 Year |
| 36 | GCS* | 50 | 15 | 35 | 10 | 40 | 40 | NR | Died of Disease < 1 Year |
| 37 | MJBV* | 80 | 16 | 64 | 16 | 4 | 0 | NR | Died of Disease < 1 Year |
| 39 | FHV* | 70 | 28 | 42 | 25 | 5 | 0 | NR | Died of Disease < 1 Year |
| | Mean | 72 | 43 | 29 | 12 | 17 | 7 | | |
| | SD | 12 | 28 | 25 | 8 | 15 | 16 | | |
| | | | | | | | | | |

| | | | | | | | | | |
|---|---|---|---|---|---|---|---|---|---|
| *Drawn from JUAREZ Hospital Experience. | | | | | | | | | |

## Claims

1. A natural cytokine mixture (NCM) including the cytokines IL-1 IL-2, IL-6, IL-8, IL-12, IFN-gamma,TNF-alpha, GM-CSF and G-CSF, for use in immunotherapy of a cancer patient in a dosage regime comprising
(i) application of said natural cytokine mixture in a skin test amount to a cancer patient such that said skin test shows either a positive or negative clinical response to said skin test within 24 hours, and
(ii) subsequent intracutaneous administration of said natural cytokine mixture to a cancer patient who shows said negative clinical response to said application in step (i).

2. Use according to claim 1, wherein 100 to 233 units of said IL-2 are employed in said administration.

3. Use according to claim 1 or 2, wherein said administration precedes surgery, with or without radiotherapy.

4. Use according to any of claims 1 to 3, wherein said administration is together with cyclophosphamide and indomethacin.

## Patentansprüche

1. Gemisch natürlicher Zytokine (GNZ), das die Zytokine IL-1, IL-2, IL-6, IL-8, II-12, IFN-gamma, TNF-alpha, GM-CSF und G-CSF enthält, für die Verwendung bei einer Immuntherapie eines Krebspatienten in einem Dosierungsplan, der Folgendes umfasst:
(i) Applikation des Gemisches natürlicher Zytokine in einer Hauttestmenge bei einem Krebspatienten derart, dass der Hauttest innerhalb von 24 Stunden entweder eine positive oder eine negative klinische Reaktion auf den Hauttest zeigt, und
(ii) anschließende intrakutane Verabreichung des Gemisches natürlicher Zytokine an einen Krebspatienten, der die negative klinische Reaktion auf die Verabreichung in Schritt (i) gezeigt hat.

2. Verwendung nach 1, wobei bei der Verabreichung 100 bis 233 Einheiten des IL-2 verwendet werden.

3. Verwendung nach 1 oder 2, wobei die Verabreichung einer Operation mit oder ohne Bestrahlungstherapie vorausgeht.

4. Verwendung nach einem der Ansprüche 1 bis 3, wobei die Verabreichung zusammen mit Cyclophosphamid und Indomethacin erfolgt.

## Revendications

1. Mélange de cytokines naturelles (MCN) comprenant les cytokines IL-1, IL-2, IL-6, IL-8, IL-12, IFN-gamma, TNF-alpha, GM-CSF et G-CSF, destiné à être utilisé en immunothérapie chez un patient souffrant d'un cancer en un schéma posologique comprenant :
(i) l'application dudit mélange de cytokines naturelles en une quantité de test cutané à un patient souffrant d'un cancer de telle sorte que ledit test cutané présente une réponse clinique positive ou négative au dit test cutané dans les 24 heures suivant l'application, et
(ii) ensuite, l'administration intradermique dudit mélange de cytokines naturelles à un patient souffrant d'un cancer qui présente ladite réponse clinique négative à ladite application dans l'étape (i).

2. Utilisation selon la revendication 1, dans laquelle 100 à 233 unités de ladite IL-2 sont utilisées dans ladite administration.

3. Utilisation selon la revendication 1 ou 2, dans laquelle ladite administration précède une opération chirurgicale, avec ou sans radiothérapie.

4. Utilisation selon l'une quelconque des revendications 1 à 3, dans laquelle ladite administration est réalisée conjointement à une administration de cyclophosphamide et d'indométhacine.
